(19) Europäisches Patentamt
European Patent Office
Office européen des brevets

(11) Veröffentlichungsnummer: **0 324 158 A2**

(12) # EUROPÄISCHE PATENTANMELDUNG

(21) Anmeldenummer: **88121613.9**

(51) Int. Cl.⁴: **C07D 213/89 , A01N 43/40**

(22) Anmeldetag: **23.12.88**

(30) Priorität: **31.12.87 DE 3744620**

(43) Veröffentlichungstag der Anmeldung:
**19.07.89 Patentblatt 89/29**

(84) Benannte Vertragsstaaten:
**AT BE CH DE ES FR GB GR IT LI NL SE**

(71) Anmelder: **BASF Aktiengesellschaft**
**Carl-Bosch-Strasse 38**
**D-6700 Ludwigshafen(DE)**

(72) Erfinder: **Zipperer, Bernhard, Dr.**
**Am Hergottsacker 6**
**D-6716 Dirmstein(DE)**
Erfinder: **Buschmann, Ernst, Dr.**
**Georg-Ludwig-Krebs-Strasse 10**
**D-6700 Ludwigshafen(DE)**
Erfinder: **Lauer, Manfred, Dr.**
**Im Zinkig 114**
**D-6700 Ludwigshafen(DE)**
Erfinder: **Ammermann, Eberhard, Dr.**
**Sachsenstrasse 3**
**D-6700 Ludwigshafen(DE)**
Erfinder: **Lorenz, Gisela, Dr.**
**Erlenweg 13**
**D-6730 Neustadt(DE)**

(54) **Pyridin-N-oxide und diese enthaltende Fungizide.**

(57) Substituierte Pyridin-N-oxide der Formel

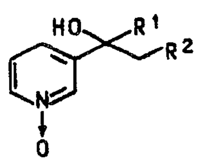

in der
$R^1$ Alkyl und
$R^2$ Phenyl oder substituiertes Phenyl bedeuten,
die pflanzenphysiologisch verträglichen Salze dieser Verbindungen und diese enthaltende Fungizide.

EP 0 324 158 A2

# Pyridin-N-oxide und diese enthaltende Fungizide

Die vorliegende Erfindung betrifft neue Pyridin-N-oxide und deren Salze sowie fungizide Mittel, die diese Verbindungen enthalten, und Verfahren zu ihrer Herstellung.

Es ist bekannt, 3-Pyridylmethanole als Fungizide zu verwenden (EP 69 330). Ihre fungizide Wirkung ist jedoch nur bei hohen Aufwandmengen ausreichend.

Es wurde nun gefunden, daß die neuen Pyridin-N-oxide der Formel

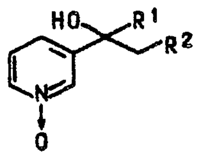

(I)

in der

$R^1$ $C_1$-$C_{12}$-Alkyl,

$R^2$ Phenyl oder substituiertes Phenyl mit 1 bis 3 Resten aus der Gruppe $C_1$-$C_4$-Alkyl, $C_1$-$C_4$-Halogenalkyl, $C_1$-$C_4$-Alkoxy, $C_1$-$C_4$-Halogenalkoxy und Halogen bedeuten, und ihre pflanzenphysiologisch verträglichen Salze sowohl eine höhere fungizide Aktivität als auch eine deutlich bessere Pflanzenverträglichkeit haben als die bekannten 3-Pyridylmethanole.

$R^1$ bedeutet beispielsweise $C_1$-$C_{12}$-Alkyl, insbesondere $C_3$-$C_8$-Alkyl, z.B. Methyl, Ethyl, n-Propyl, iso-Propyl, n-Butyl, iso-Butyl, tert.-Butyl, n-Pentyl, iso-Pentyl, neo-Pentyl, n-Hexyl, neo-Hexyl, n-Heptyl, n-Octyl, n-Nonyl, n-Decyl, n-Undecyl, n-Dodecyl.

$R^2$ bedeutet beispielsweise Phenyl, Mono-, Di- oder Trimethylphenyl, Isopropylphenyl, tert.-Butylphenyl, Trifluormethylphenyl, Pentafluorethylphenyl, Mono-, Di- oder Trimethoxyphenyl, tert.-Butoxyphenyl, Tetrafluorethoxyphenyl, Fluorphenyl, Chlorfluorphenyl, Mono-, Di- oder Trichlorphenyl.

Salze sind z.B. die Säuureadditionssalze, beispielsweise die Salze von anorganischen Säuren, z.B. Chlorwasserstoffsäure, Bromwasserstoffsäure, Schwefelsäure, Phosphorsäure, oder von organischen Säuren, z.B. $C_1$-$C_4$-Alkylcarbonsäuren, Essigsäure, Propionsäure, Tolylsulfonsäure, Dodecylbenzolsulfonsäure.

Die Verbindungen enthalten mindestens ein asymmetrisches C-Atom und treten daher in Form ihrer optischen Isomeren, Enantiomeren, auf. Die Erfindung umfaßt sowohl die reinen Enantiomeren als auch die Racemate und die Diastereomeren.

Die Herstellung der Pyridin-N-oxide der Formel I kann z.B. in der Weise erfolgen, daß man ein 3-substituiertes Pyridin der Formel

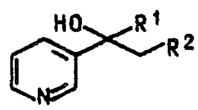

(II)

in der $R^1$ und $R^2$ die oben genannten Bedeutungen haben, nach üblichen Methoden oxidiert. Als Oxidationsmittel eignen sich beispielsweise Persäuren oder in situ aus Wasserstoffperoxid und Carbonsäureanhydriden erzeugte Persäuren, Wasserstoffperoxid oder organische Derivate des Wasserstoffperoxids (vgl. z. B. R. A. Abramovitch und E. M. Smith in Chemistry of Heterocyclic Compounds, Band 14, Suppl. 2, Seite 1 ff., John Wiley, New York 1974; A. R. Katritzky, J. M. Lagowski, The Chemistry of Heterocyclic N-Oxides, S. 21 ff, Academic Press, New York 1971). Ein für dieses Verfahren besonders vorteilhaftes Oxidationsmittel ist 3-Chlorperbenzoesäure (vgl. J. C. Craig, K. K. Purnshothaman, J. Org. Chem. 35, 1721 (1970)). Als Verdünnungsmittel zur Durchführung der Oxidation mit 3-Chlorperbenzoesäure verwendet man beispielsweise chlorierte Kohlenwasserstoffe, insbesondere Dichlormethan oder Trichlormethan.

3-Pyridylcarbinole der Formel II sind bekannt aus EP-A-69 330. Die dort gegebenen Herstellungsvorschriften können ohne Schwierigkeiten auch auf andere Alkyl- oder Aryl-Reste übertragen werden.

Die folgenden Beispiele erläutern die Herstellung der neuen Pyridin-N-oxide.

Beispiel 1

1-(2,4-Dichlorphenyl)-2-(pyridin-1-oxid-3-yl)-2-decanol

Zu einer Lösung von 8,5 g (0,022 mol) 1-(2,4-Dichlorphenyl)-2-(pyridin-3-yl)-2-decanol in 100 ml Dichlormethan tropft man unter Rühren die Lösung von 7,6 g (0,044 mol) 3-Chlor-perbenzoesäure in 60 ml Dichlormethan. Man rührt über Nacht, wäscht die Reaktionslösung zweimal mit 5 % (Gew.%) Natronlauge und einmal mit Wasser, trocknet über Natriumsulfat und engt i. Vak. ein. Der ölige Rückstand wird mit Diisopropylether bis zur Kristallisation verrührt. Nach Absaugen und Trocknen i. Vak. erhält man 6,8 g beigefarbenes Pulver vom Schmp. 99-102° C (78 % d. Th) (Verbindung Nr. 1).

Beispiel 2

1-(4-Fluorphenyl)-2-(pyridin-1-oxid-3-yl)-2-decanol

Zu einer Lösung von 7,5 g (0,022 mol) 1-(4-Fluorphenyl)-2-(pyridin-3-yl)-2-decanol in 100 ml Dichlormethan tropft man unter Rühren die Lösung von 7,6 g (0,044 mol) 3-Chlorperbenzoesäure in 60 ml Dichlormethan. Die über Nacht gerührte Reaktionslösung wird auf etwa die Hälfte ihres Volumens eingeengt und auf eine Säule mit ca. 300 g basischem Aluminiumoxid (Aktivitätsstufe I) aufgetragen. Unpolare Verunreinigungen werden mit Dichlormethan, das Pyridin-N-oxid mit Dichlormethan/Methanol (3:1) eluiert. Nach Einengen i. Vak. erhält man 5,5 g (70 % d. Th) Verbindung Nr. 2 als gelbes Öl. $^1H$-NMR des Pyridinteils (200 MHz, $CDCl_3$): $\delta$ = 8,30 (2-H), 8,05 (6-H), 7,20 (4-H, 5-H).

Entsprechend können folgende Verbindungen hergestellt werden:

Tabelle 1

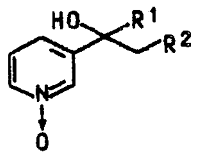

(I)

| Verbindung Nummer | $R^1$ | $R^2$ | Fp (°C) |
|---|---|---|---|
| 1 | n-Octyl | 2,4-Dichlorphenyl | 99-102 |
| 2 | n-Octyl | 4-Fluorphenyl | Öl |
| 3 | iso-Propyl | 4-Chlorphenyl | |
| 4 | iso-Propyl | 2-Chlorphenyl | |
| 5 | iso-Propyl | 2,4-Dichlorphenyl | 150-151 |

Tabelle 1 (Fortsetzung)

| Verbindung Nummer | $R^1$ | $R^2$ | Fp (°C) |
|---|---|---|---|
| 6 | n-Propyl | 2,4-Dichlorphenyl | |
| 7 | n-Propyl | Phenyl | |
| 8 | n-Propyl | 4-Methylphenyl | |
| 9 | n-Butyl | 2,4-Dichlorphenyl | |
| 10 | n-Butyl | 4-Chlorphenyl | |
| 11 | iso-Butyl | 2,4-Dichlorphenyl | 161 |
| 12 | iso-Butyl | 4-Chlorphenyl | |
| 13 | iso-Butyl | 4-Fluorphenyl | |
| 14 | iso-Butyl | 3-Trifluormethylphenyl | |
| 15 | iso-Butyl | 2-Chlorphenyl | |
| 16 | n-Pentyl | 4-Chlorphenyl | |
| 17 | n-Pentyl | 2,4-Dichlorphenyl | |
| 18 | n-Pentyl | 2-Chlorphenyl | |
| 19 | neo-Hexyl | 2-Chlorphenyl | |
| 20 | neo-Hexyl | 4-Chlorphenyl | |
| 21 | neo-Hexyl | 2,4-Dichlorphenyl | 150-153 |
| 22 | neo-Hexyl | 4-Trifluormethylphenyl | |
| 23 | neo-Hexyl | 3-Trifluormethylphenyl | |
| 24 | neo-Hexyl | 4-Fluorphenyl | |
| 25 | neo-Hexyl | 4-tert.-Butylphenyl | |
| 26 | neo-Hexyl | 4-Chlor-3-trifluormethylphenyl | |
| 27 | n-Hexyl | 2,4-Dichlorphenyl | |
| 28 | n-Heptyl | 2,4-Dichlorphenyl | |
| 29 | n-Octyl | 2-Chlorphenyl | 65-67 |
| 30 | n-Octyl | 4-Chlorphenyl | Öl |
| 31 | n-Octyl | 3-Trifluormethylphenyl | Öl |
| 32 | n-Nonyl | 2,4-Dichlorphenyl | |
| 33 | n-Decyl | 2,4-Dichlorphenyl | |
| 34 | n-Undecyl | 2,4-Dichlorphenyl | |
| 35 | n-Dodecyl | 2,4-Dichlorphenyl | |
| 36 | n-Dodecyl | 4-Chlorphenyl | |
| 37 | n-Dodecyl | 2-Chlorphenyl | 57-64 |
| 38 | n-Dodecyl | 4-Fluorphenyl | 73-76 |
| 39 | neo-Pentyl | 2,4-Dichlorphenyl | |
| 40 | neo-Pentyl | 4-Chlorphenyl | |
| 41 | neo-Pentyl | 2-Chlorphenyl | |
| 42 | neo-Pentyl | 4-Fluorphenyl | |

Tabelle 1 (Fortsetzung)

| Verbindung Nummer | $R^1$ | $R^2$ | Fp (°C) |
|---|---|---|---|
| 43 | neo-Pentyl | 2-Fluorphenyl | |
| 44 | neo-Pentyl | 4-Trifluormethylphenyl | |
| 45 | neo-Pentyl | 3-Trifluormethylphenyl | |
| 46 | neo-Pentyl | 4-(4-tert.-Butylphenyl) | |

| Verbindung Nummer | NMR-Daten und IR-Werte |
|---|---|
| 2 | $^1$H-NMR ($CDCl_3$): δ = 8.30 (s,2'-H), 8.05 (s,6'-H), 7.20 (br.m, 4'-H,5'-H), 7.10 (dd, $2H_o$), 6.85 (dd, $2H_m$), 4.75 (br.s, OH), 3.03 (dd, 1-$CH_2$), 1.80 (br.m, 3-$CH_2$), 1.20 (br.m, 4- bis 9-$CH_2$), 0.85 (t, 10-$CH_3$). IR (Film): ν = 2953, 2927, 2855, 1601, 1509, 1426, 1298, 1261, 1223, 1158, 1015 $cm^{-1}$. |
| 30 | $^1$H-NMR ($CDCl_3$): δ = 8.30 (s, 2'-H), 8.05 (s, 6'-H), 7.20 (br.m, 4'-H, 5'-H), 7.15 (d, $2H_o$), 6.95 (d, $2H_m$), 4.25 (br.s, OH), 3.00 (dd, 1-$CH_2$), 1.78 (br.m, 3-$CH_2$), 1.20 (br.m, 4- bis 9-$CH_2$), 0.85 (t, 10-$CH_3$). IR (Film): ν = 3223, 2927, 2854, 1602, 1490, 1467, 1428, 1258, 1149, 1093, 1016 $cm^{-1}$. |
| 31 | $^1$H-NMR ($CDCl_3$): δ = 8.30 (s, 2'-H), 8.05 (s, 6'-H), 7.40 bis 7.15 (m, 4'-H, 5'-H, $2H_o$, $H_m$, $H_p$), 4.65 (br.s, OH), 3.10 (dd, 1-$CH_2$), 1.80 (br.m, 3-$CH_2$), 1.20 (br.m, 4- bis 9-$CH_2$), 0.85 (t, 10-$CH_3$). IR (Film): ν = 3220, 2928, 2856, 1602, 1428, 1333, 1259, 1204, 1163, 1124, 1096, 1075 $cm^{-1}$. |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,

Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen.
Plasmopara viticola an Reben,
Alternaria-Arten an Obst und Gemüse.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz eingesetzt werden, z.B. gegen Paecilomyces variotii.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweiise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. 5 mit 10 Gew.-Teilen N-Methyl-α-pyrrolidon und erhält eine Lösung, die zur Anwendung in Form kleinster Tropfen geeignet ist.

II. 20 Gew.-Teile der Verbindung Nr. 5 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 5 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 5 werden in einer Mischung gelöst, die aus 25 Gew.-Teilen Cyclohexanol, 65 Gew.-Teilen einer Mineralölfraktion vom Siedepunkt 210 bis 280 °C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 5 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 5 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 5 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 5 werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 5 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise:

Schwefel,
Dithiocarbamate und deren Derivate, wie
Ferridimethyldithiocarbamat,
Zinkdimethyldithiocarbamat,
Zinkethylenbisdithiocarbamat,
Manganethylenbisdithiocarbamat,
Mangan-Zink-ethylendiamin-bis-dithiocarbamat,
Tetramethylthiuramdisulfide,
Ammoniak-Komplex von Zink-(N,N-ethylen-bis-dithiocarbamat),
Ammoniak-Komplex von Zink-(N,N′-propylen-bis-dithiocarbamat),
Zink-(N,N′-propylen-bis-dithiocarbamat),
N,N′-Polypropylen-bis-(thiocarbamoyl)-disulfid;

Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat;
5-Nitro-isophthalsäure-di-isopropylester

heterocyclische Substanzen, wie
2-Heptadecyl-2-imidazolin-acetat,
2,4-Dichlor-6-(o-chloranilino)-s-triazin,
0,0-Diethyl-phthalimidophosphonothioat,
5-Amino-1-[bis-(dimethylamino)-phosphinyl]-3-phenyl-1,2,4-triazol,
2,3-Dicyano-1,4-dithioanthrachinon,
2-Thio-1,3-dithio-(4,5-b)-chinoxalin,
1-(Butylcarbamoyl)-2-benzimidazol-carbaminsäuremethylester,
2-Methoxycarbonylamino-benzimidazol,
2-(Furyl-(2))-benzimidazol,
2-(Thiazolyl-(4))-benzimidazol,
N-(1,1,2,2-Tetrachlorethylthio)-tetrahydrophthalimid,
N-Trichlormethylthio-tetrahydrophthalimid,
N-Trichlormethylthio-phthalimid,

N-Dichlorfluormethylthio-N',N'-dimethyl-N-phenyl-schwefelsäurediamid,
5-Ethoxy-3-trichlormethyl-1,2,3-thiadiazol,
2-Rhodanmethylthiobenzthiazol,
1,4-Dichlor-2,5-dimethoxybenzol,
4-(2-Chlorphenylhydroazano)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin,
2,3-Dihydro-5-carboxanilido-6-methyl-1,4-oxathiin-4,4-dioxid,
2-Methyl-5,6-dihydro-4H-pyran-3-carbonsäure-anilin,
2-Methyl-furan-3-carbonsäureanilid,
2,5-Dimethyl-furan-3-carbonsäureanilid,
2,4,5-Trimethyl-furan-3-carbonsäureanilid,
2,5-Dimethyl-furan-3-carbonsäurecyclohexylamid,
N-Cyclohexyl-N-methoxy-2,5-dimethyl-furan-3-carbonsäureamid,
2-Methyl-benzoesäure-anilid,
2-Iod-benzoesäure-anilid,
N-Formyl-N-morpholin-2,2,2-trichlorethylacetal,
Piperazin-1,4-diylbis-(1-(2,2,2-trichlor-ethyl)-formamid,
1-(3,4-Dichloranilino)-1-formylamino-2,2,2-trichlorethan ,
2,6-Dimethyl-N-tridecyl-morpholin bzw. dessen Salze,
2,6-Dimethyl-N-cyclodedecyl-morpholin bzw. dessen Salze,
N-[3-(p-tert.-Butylphenyl)-2-methylpropyl]-cis-2,6-dimethylmorpholin,
N-[3-(p-tert.-Butylphenyl)-2-methylpropyl]-piperidin,
1-[2-(2,4-Dichlorphenyl)-4-ethyl-1,3-dioxolan-2-yl-ethyl]-1H-1,2,4-triazol
1-[2-(2,4-Dichlorphenyl)-4-n-propyl-1,3-dioxolan-2-yl-ethyl]-1H-1,2,4-triazol,
N-(n-Propyl)-N-(2,4,6-trichlorphenoxyethyl)-N'-imidazol-yl-harnstoff,
1-(4-Chlorphenoxy)-3,3-dimethyl-1-(1H-1,2,4-triazol-1-yl)-2-butanon,
1-(4-Chlorphenoxy)-3,3-dimethyl-1-(1H-1,2,4-triazol-1-yl)-2-butanol,
α-(2-Chlorphenyl)-α-(4-chlorphenyl)-5-pyrimidin-methanol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol,
sowie verschiedene Fungizide, wie
Dodecylguanidinacetat,
3-[3-(3,5-Dimethyl-2-oxycyclohexyl)-2-hydroxyethyl]-glutarimid,
Hexachlorbenzol,
DL-Methyl-N-(2,6-dimethyl-phenyl)-N-furoyl(2)-alaninat,
DL-N-(2,6-Dimethyl-phenyl)-N-(2'-methoxyacetyl)-alanin-methylester,
N-(2,6-Dimethylphenyl)-N-chloracetyl-D,L-2-aminobutyrolacton,
DL-N-(2,6-Dimethylphenyl)-N-(phenylacetyl)-alaninmethylester,
5-Methyl-5-vinyl-3-(3,5-dichlorphenyl)-2,4-dioxo-1,3-oxazolidin,
3-[3,5-Dichlorphenyl(-5-methyl-5-methoxymethyl]-1,3-oxazolidin-2,4-dion,
3-(3,5-Dichlorphenyl)-1-isopropylcarbamoylhydantoin,
N-(3,5-Dichlorphenyl)-1,2-dimethylcyclopropan-1,2-dicarbonsäureimid,
2-Cyano-[N-(ethylaminocarbonyl)-2-methoximino]-acetamid,
1-[2-(2,4-Dichlorphenyl)-pentyl]-1H-1,2,4-triazol,
2,4-Difluor-α-(1H-1,2,4-triazolyl-1-methyl)-benzhydrylalkohol,
N-(3-Chlor-2,6-dinitro-4-trifluormethyl-phenyl)-5-trifluormethyl-3--chlor-2-aminopyridin,
1-((bis-(4-Fluorphenyl)-methylsilyl)-methyl)-1H-1,2,4-triazol.

Anwendungsbeispiele

Als Vergleichswirkstoff wurde 1-(2,4-Dichlorphenyl)-2-(pyridin-3-yl)-3-methyl-2-butanol (A) - bekannt aus EP-69330 - benutzt.

Anwendungsbeispiel

Wirksamkeit gegen Botrytis cinerea

Kleinparzellen von Freiland-Erdbeerpflanzen der Sorte "Senga-Sengana" wurden zum Zeitpunkt der Hauptblüte mit einer wäßrigen Suspension, die 80 % (Gew.%) Wirkstoff und 20 % Dispergiermittel in der Trockensubstanz enthält, tropfnaß gespritzt. 2 Tage später wurde die gesamte Anlage mit einer Sporensuspension von Botrytis cinerea inokuliert und nach weiteren 5 Tagen wurden die entsprechenden Parzellen nochmals behandelt. 38 Tage danach wurden die reifen Früchte auf den Botrytis-Befall durchgesehen.

Das Ergebnis zeigt, daß der Wirkstoff 5 bei einer Aufwandmenge von 2 kg Wirkstoff/ha eine bessere fungizide Wirkung zeigt (75 %) als der bekannte Vergleichswirkstoff A (18 %).

## Ansprüche

1. 3-substituiertes Pyridin-N-oxid der Formel

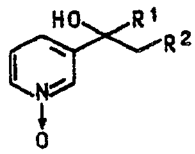

(I)

in der
$R^1$ $C_1$-$C_{12}$-Alkyl und
$R^2$ Phenyl oder substituiertes Phenyl mit 1 bis 3 Resten aus der Gruppe $C_1$-$C_4$-Alkyl, $C_1$-$C_4$-Halogenalkyl, $C_1$-$C_4$-Alkoxy, $C_1$-$C_4$-Halogenalkoxy und Halogen bedeuten,
und die pflanzenphysiologisch verträglichen Salze dieser Verbindung.

2. Verfahren zur Herstellung eines Pyridin-N-oxids der Formel

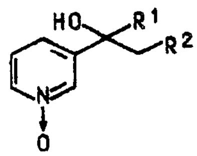

(I)

in der
$R^1$ $C_1$-$C_{12}$-Alkyl und
$R^2$ Phenyl oder substituiertes Phenyl mit 1 bis 3 Resten aus der Gruppe $C_1$-$C_4$-Alkyl, $C_1$-$C_4$-Halogenalkyl, $C_1$-$C_4$-Alkoxy, $C_1$-$C_4$-Halogenalkoxy und Halogen bedeuten,
dadurch gekennzeichnet, daß man ein Pyridylcarbinol der Formel II,

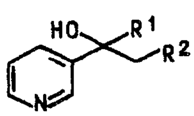

(II)

in der $R^1$ und $R^2$ die oben genannten Bedeutungen haben, mit einem Oxidationsmittel umsetzt.

3. Fungizides Mittel, enthaltend einen inerten Trägerstoff und ein 3-substituiertes Pyridin-N-oxid der Formel

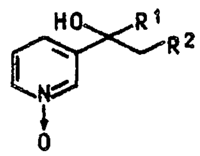

(I)

in der
$R^1$ $C_1$-$C_{12}$-Alkyl und
$R^2$ Phenyl oder substituiertes Phenyl mit durch 1 bis 3 Resten aus der Gruppe $C_1$-$C_4$-Alkyl, $C_1$-$C_4$-Halogenalkyl, $C_1$-$C_4$-Alkoxy, $C_1$-$C_4$-Halogenalkoxy und Halogen bedeuten,
oder ein pflanzenphysiologisch verträgliches Salz dieser Verbindung.

4. Verfahren zur Bekämpfung von Pilzen, dadurch gekennzeichnet, daß man die Pilze oder die vom Pilzbefall bedrohten Materialien, Pflanzen, Böden oder Saatgüter behandelt mit einer fungizid wirksamen Menge eines 3-substituierten Pyridin-N-oxids der Formel

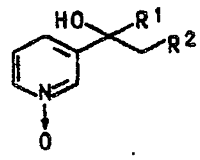

(I)

in der
$R^1$ $C_1$-$C_{12}$-Alkyl und
$R^2$ Phenyl oder substituiertes Phenyl mit 1 bis 3 Resten aus der Gruppe $C_1$-$C_4$-Alkyl, $C_1$-$C_4$-Halogenalkyl, $C_1$-$C_4$-Alkoxy, $C_1$-$C_4$-Halogenalkoxy und Halogen bedeuten,
oder eines pflanzenphysiologisch verträglichen Salzes dieser Verbindung.

5. Verbindung gemäß Anspruch 1, dadurch gekennzeichnet, daß $R^1$ iso-Propyl und $R^2$ 2,4-Dichlorphenyl bedeuten.

6. Verbindung gemäß Anspruch 1, dadurch gekennzeichnet, daß $R^1$ iso-Butylund $R^2$ 2,4-Dichlorphenyl bedeuten.